# EUROPEAN PATENT APPLICATION

(11) **EP 4 516 094 A1**
(43) Date of publication of application: **05.03.2025**
(21) Application number: 23382879.7
(22) Date of filing: 29.08.2023
(51) Int. Cl.: A01K 1/03, A01K 29/00, A61N 5/01, A61N 5/06

(54) **DEVICE FOR AUTOMATED LIGHT STIMULATION OF RODENTS**

(71) Applicant: Universidad Antonio de Nebrija, 28240 Hoyo de Manzanares (Madrid) (ES); Universidad de Oviedo, 33003 Oviedo (ES)
(72) Inventor: ARIAS DEL CASTILLO, Natalia, 28240 HOYO DE MANZANARES (Madrid) (ES); OLAZAGOITIA RODRIGUEZ, Jose Luis, 28240 HOYO DE MANZANARES (Madrid) (ES); AVILES LOPEZ, Alejandro, 28240 HOYO DE MANZANARES (Madrid) (ES); ARIAS PEREZ, Jorge L., 280240 HOYO DE MANZANARES (Madrid) (ES)
(74) Representative: Herrero & Asociados, S.L.

(57) **Abstract**

Automated device for light stimulation of rodents comprising a closed enclosure with a transparent base (2), a light emitter (3) in the lower part of the enclosure, a mechanism for moving (4) the light emitter (3) in the X-Y plane to provide the desired stimulation to the rodent, a system for capturing the image and movement (5) of the rodent, a data processor (6) and a controller (7) to send signals to the mechanism of movement in such a way that the emitter (3) is always in a desired position depending on the position of the rodent.

## Description

### TECHNICAL FIELD

The present invention falls within the field of animal experimentation. More particularly, it relates to a device for automatically stimulating rodents by means of light.

### STATE OF THE ART

Different studies have shown that the action of photobiomodulation on the brain causes protective effects in traumatic brain damage [Chen, A.C. et al., 2011. PLoS ONE 6, e22453], ischemic stroke [Lev, N et al., 2008. Neurotoxicology 29, 397-405], Alzheimer's disease [Hamblin MR, et al., 2021. J Alzheimers Dis. 83(4):1395-1397] and in psychological disorders such as depression or anxiety [Eshaghi E, et al., 2019. Lasers Surg Med. 51(7):634-642].

Specifically, the application of photobiomodulation implies the improvement of mitochondrial function [Hamblin, M.R. 2018. Photochem. Photobiol. 94, 199-212], which stimulates the photodissociation of nitric oxide, increasing its bioavailability as a signaling molecule and at the same time enabling the ability of the enzyme, cytochrome C oxidase, to bind molecular oxygen, thus improving electron flow through the electron transport chain and the production of energy, in the form of ATP (adenosine triphosphate) [Kaynezhad, P et al., 2016. Exp. Eye Res.152, 88-93.31]. Likewise, photobiomodulation with near infrared light induces a moderate increase in the production of reactive oxygen species without increasing oxidative stress [Amaroli, Aet al.,2019. Lasers Med. Sci. 34, 495-504] and indeed reduces reactive oxygen species in oxidatively stressed neurons [Huang, Y.-Y., et al., 2013. J. Biophotonics 6, 829- 838]. This modest generation of reactive oxygen species can activate redox-sensitive transcription factors and initiate upregulation of antioxidant, detoxification, and survival pathways [Chen, A.C. et al., 2011. PLoS ONE 6, e22453; Lev, N et al., 2008. Neurotoxicology 29, 397-405].

Therefore, a device is desirable that allows the application of near-infrared light automatically to a plurality of subjects at the same time and in accordance with the regional and European ethical regulations for the use of animals in experimentation.

### SUMMARY OF THE INVENTION:

The object of the present invention is an automatic device for the detection and monitoring of laboratory animals within a limited enclosure for the application of a treatment based on light whose wavelength is between 510 and 1100 nm.

The device consists mainly of an enclosure that is closed on all sides except the upper part and wherein the base of the enclosure is transparent, a system for capturing the image and movement of the animal at all times and that covers the entire enclosure, a processor for collecting and analyzing the data provided by the capture system, an electromagnetic wave emitter to provide the required treatment to the animal and means of movement of the wave emitter in the X-Y plane, which move the emitter depending on the position of the rodent, previously identified by the capture system and sent to the means of movement by a controller. In a particular implementation, the processor and the controller may be in the same device.

The wave emitter is preferably a laser or LED emitter with a wavelength between 510-1100 nm (10-80 Hz).

The enclosure where the animal or animals to be monitored are housed will have an area of 500 to 800 cm2. The standard measurements are, according to the European regulation, 800 cm2 for rats (European type IIIH) and 530 cm2 (European type IIL).

### BRIEF DESCRIPTION OF FIGURES

In order to help a better understanding of the features of the invention and to complement this description, the following figures are attached as an integral part thereof, the nature of which is illustrative and not limiting:
Figure 1 shows the essential elements of the invention.
Figure 2 shows a side view of the invention.

### DETAILED DESCRIPTION

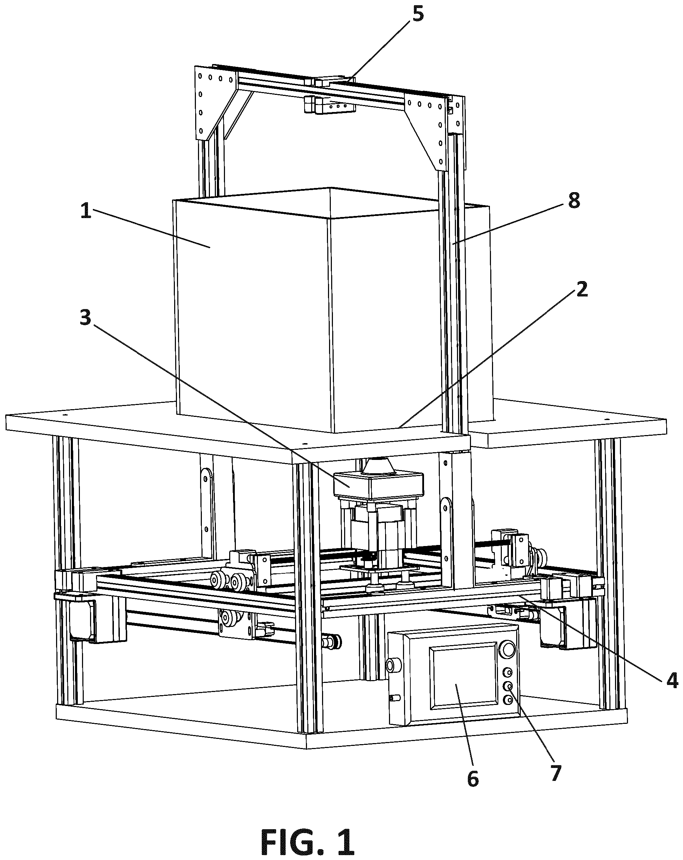

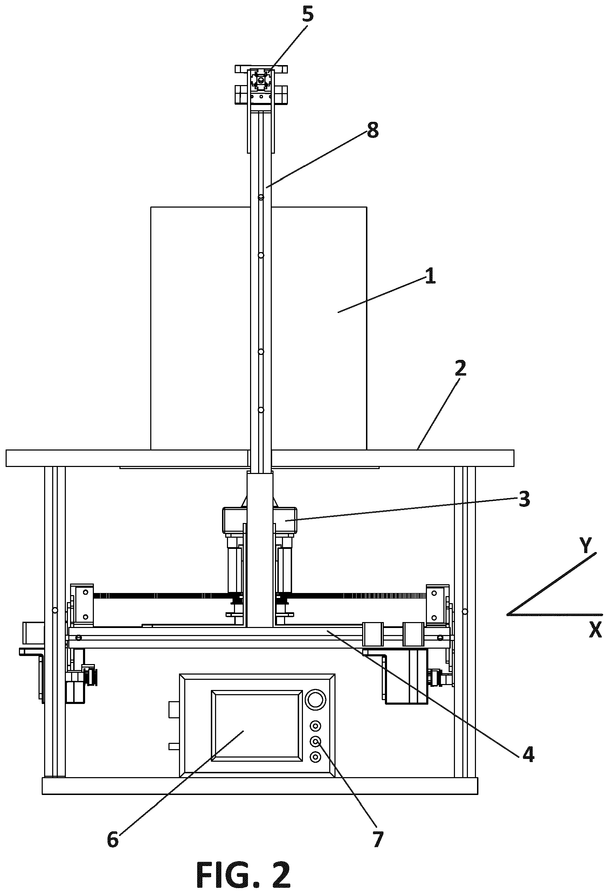

With reference to figures 1-2, the enclosure 1 can have, in a preferred example, a rectangular, circular or oval shape. Its walls can be opaque, translucent or transparent, as long as the base 2 is transparent to the wavelength of the emitter 3 (transparent base or made of sufficiently separated or fine grids). The side walls have a minimum height of 20cm.

The translation means 4 of the emitter 3 comprise, in a particular implementation, at least two motors that act on a pulley that transmits the movement through belts and guided in the X-Y plane.

The laboratory animal is introduced into the enclosure and a prudential time is allowed to elapse for it to acclimate to the new space. The capture system 5 detects the animal within the enclosure. Processor 6 receives the data from the capture system and runs a program that analyzes the image through artificial vision techniques or simply color analysis techniques and returns the position of the animal in X and Y coordinates within the enclosure. This information is passed to the controller 7, which sends the movement signal to the means of movement 4 of the emitter, so that the motors act on the X and Y axes, positioning the emitter 3 in the desired coordinates, just below the animal. Once correctly positioned, it is confirmed that the position of the animal and the wave emitter coincide within a range (+/- 2 mm of the target X-Y position) and treatment is applied to the animal by activating the emitter (the controller sends the activation signal to the emitter, which remains active for the time necessary for the treatment of the animal, or until the animal changes position).

The enclosure 1, in its most basic version, can be configured as a square, rectangular or circular area, although the only requirement is that its base 2 be flat, so that its walls could have any shape. The area where the laboratory animal is located is completely covered by the translation means. The position and movement capture system 5 (a camera, for example) can be located at the indicated height through some adapters 8 attached to the walls of the enclosure that allow it to be located at the correct distance.

The animal capture system 5 can be based on different technologies, such as visible spectrum cameras to detect the movement and location of the laboratory animal. In its simplest embodiment, it would suffice to place a visible spectrum camera in the upper position of the closed enclosure, so that its viewing area covers the entire movement area of the laboratory animal. The positioning of the camera, the sensor or the sensors used can be any, as long as its working or detection range covers the entire movement area of the laboratory animal. In a particular implementation, the camera is a camera in the visible spectrum that will preferably be positioned on the vertical of the center of the area of the enclosure at a height of at least 25 cm above the base 2; in this way it is ensured that the entire base of the enclosure on which the animal will move is covered (taking into account, for example, the low-cost Raspberry Pi v2 camera, which is an 8 MP module with a 1/4" CMOS sensor, 3.04mm focal length and viewing angles of 62.2° (horizontal) and 48.8° (vertical). The Raspberry Pi HQ camera is a 12.3 MP module with a 1/2.3" Sony IMX477 sensor and supports interchangeable lenses, assuming a 6mm lens, the diagonal angle of view is 69.40°. For a 12" x 12" enclosure, the height required from the vertical of the center of the square is approximately 8.5" for the v2 camera and 8.5" for the HQ camera).

The processor 6 is in charge of analyzing the data obtained by the capture system 5 and obtaining the exact position of the animal in the area. In the previous example, the processor can be a 4Gb Raspberry Pi 4, but this can vary to a common PC computer, or a dedicated electronic board. It can be based on a computer, an acquisition system based on SBC (Single Board Computer) type development boards, Arduino or similar, on FPGAs, or on electronic boards programmed for this purpose. In another implementation, the processor and controller are one, for example a controller based on an SBC, capable of managing the data obtained from the capture system 5 and with sufficient computing power to analyze (in real time or not) the animal position. The controller, once the position of the animal has been determined, will send to the means of movement the coordinates to which the wave emitter should move.

The translation means 4 of the light emitter 3 make it possible to cover the entire area where the animal evolves and thus position the emitter where it is needed. The means of movement can be based on different technologies. One of the simplest embodiments would be that of a system of guides in X and Y that allow the emitter to be moved in both axes, driven by electric motors that transmit the movement through toothed belts, or through a system of endless screw. X and Y coordinates are understood to be those of a plane parallel to the base of enclosure 2.

The time of application of the treatment, frequency, type of wave or light, accumulation of treatment, etc. is determined by controller 7.

The invention is suitable for applying the treatment to one or several laboratory animals at the same time or individually. In the event of having to treat several animals in the same movement area, the animals will be uniquely identified in an appropriate way so that the capture system can identify the status of each and every one of the individuals at all times. The controller 7 itself will decide the order of application of the treatment based on the needs previously established by the researcher for each subject.

The invention allows the administration of light stimulation at different wavelengths, at the request of the researcher. Likewise, it prevents the manipulation of rodent until the stimulation cycle has ended, thus avoiding biases caused by the environment, the researcher or the administration conditions, thanks to the fact that everything is automated and standardized for all subjects equally. All this also reduces the levels of stress, due to manipulation, isolation or temporary retention of the experimental subjects.

Lastly, the device facilitates the work of the researcher in several ways: firstly, the administration tasks are usually very long as a large number of animals are passed, one by one, under this stimulation; which usually entails, on the one hand, the use of many different materials, and on the other hand, a great demand on researchers during the application of the stimulation, as it is necessary to pay attention to many different factors.

In summary, some of the advantages of the device of the invention are the following:
- Wavelengths adjustable to the needs of the treatment;
- Exact automatic control of the place and time of application of the desired light stimulation through the automated tracking of the subject;
- Adaptable to the application of light stimulation to any part of the subject, such as the skull, abdominal area, tissue, etc.;
- Avoids biases due to researcher manipulation;
- Reduces the stress levels of the animals by avoiding experimental manipulation;
- The stimulation is applied in an environment to which the subjects are accustomed;
- Within the group of experimental subjects introduced into the device, the treatment can be directed individually to each one of them.

In view of this description and figures, the person skilled in the art will be able to understand that the invention has been described according to some preferred embodiments thereof, but that multiple variations can be introduced in said preferred embodiments, without exceeding the object of the invention as claimed.

## Claims

1. Device for automated light stimulation of rodents comprising:
- an enclosure (1) closed except in its upper part, the enclosure being transparent to electromagnetic waves at its lower base (2);
- a light emitter (3) in the lower part of the enclosure;
- translation means (4) of the light emitter (3) in the X-Y plane parallel to the base (2), to provide the desired stimulation to the rodent in a determined position;
- a system for capturing the image and movement (5) of the rodent capable of covering the entire volume of the enclosure (1) and providing data on the position of the rodent;
- A processor (6) of the data obtained on the position of the rodent and a controller (7) to send signals to the means of movement (4) of the emitter so that the emitter (3) is always in the position determined based on of the data received by the processor about the position of the rodent.

2. Device according to claim 1, wherein the light emitter has a wavelength between 510-1100 nm, the emission source being a laser or LED.

3. Device according to any of the preceding claims, wherein the enclosure has dimensions of up to 800 cm2 for rats and 530 cm2 for mice.
